# EUROPEAN PATENT SPECIFICATION

(11) **EP 4 609 818 B1**
(45) Date of publication and mention of the grant of the patent: **26.11.2025**
(21) Application number: 24160185.5
(22) Date of filing: 28.02.2024
(51) Int. Cl.: A61B 34/32, A61B 17/28, A61B 34/37, A61F 9/007, A61B 17/00, A61B 90/00

(54) **SURGICAL ROBOTIC SYSTEM AND CONTROL OF SURGICAL ROBOTIC SYSTEM**
CHIRURGISCHES ROBOTERSYSTEM UND STEUERUNG DES CHIRURGISCHEN ROBOTERSYSTEMS
SYSTÈME ROBOTIQUE CHIRURGICAL ET COMMANDE DE SYSTÈME ROBOTIQUE CHIRURGICAL

(43) Date of publication of application: 03.09.2025
(73) Proprietor: Carl Zeiss Meditec AG, 07745 Jena (DE)
(72) Inventor: HILLENBRAND, Matthias, 73447 Oberkochen (DE); BRIEL, Marius, 76344 Eggenstein-Leopoldshafen (DE); HAIDE, Ludwig, 76344 Eggenstein-Leopoldshafen (DE); VOIGT, Christian, 73447 Oberkochen (DE); BEELEN, Maarten, 5612 AP Eindhoven (NL); SMIT, Jorrit, 5612 AP Eindhoven (NL)
(74) Representative: DeltaPatents B.V.

(56) References cited:
- EP-A1- 4 104 779
- WO-A1-2016/030336
- US-A1- 2019 307 523
- US-A1- 2021 228 284
- US-A1- 2022 265 383
- US-B2- 9 907 696

## Description

### TECHNICAL FIELD

The invention relates to a surgical robotic system for use in an intraocular procedure. The invention further relates to a computer program product comprising instructions to perform the method.

### BACKGROUND

Intraocular surgical procedures increasingly involve the use of surgical robotic systems. Rather than operating entirely autonomously, such surgical robotic systems are expected for the foreseeable future to remain at least in part under the control of a human operator. For example, the human operator may directly or indirectly control the movement of a surgical instrument mounted to a surgical arm of the surgical robotic system. Nevertheless, it is expected that future surgical robotic systems may be more autonomous and require less or even no involvement of a human operator.

A surgical robotic system designed for intraocular procedures may be provided with a surgical arm which comprises a movable arm part, with the movable arm part comprising an end effector for holding a surgical instrument. Accordingly, the surgical instrument may be positioned by the surgical arm. An actuator subsystem may be provided for actuating the movable arm part to control a pose of the surgical instrument. Here, the term 'pose' may refer to a position and orientation of surgical instrument. Through such actuation, the surgical instrument may be moved in a lateral direction, for example relative to a retinal surface, and in a longitudinal direction along a longitudinal axis of the surgical instrument. Longitudinal movement may typically allow the surgical instrument to be moved towards and away from a surgical target within an interior of the eye. Accordingly, the surgical instrument may be used to modify (biological) tissue near the surgical target, to deliver an agent to the surgical target, etc. Examples of surgical instruments include, but are not limited to, forceps, mechanical cutters, coagulation cutters, scissors, injection needles, sealing devices, etc.

Surgical robotic systems of the above type are known per se. For example, WO 2016/030336 A1 describes a surgical robotic system for use in a surgical procedure, comprising a surgical arm comprising a movable arm part, the movable arm part comprising an instrument connector for mounting of a surgical instrument, the surgical instrument having a longitudinal axis, the movable arm part having at least one degree-of-freedom to enable longitudinal movement of the surgical instrument along the longitudinal axis of the surgical instrument towards a surgical target. The surgical robotic system is said to further comprise a human machine interface for receiving positioning commands from a human operator for controlling the longitudinal movement of the surgical instrument, an actuator configured for actuating the movable arm part to effect the longitudinal movement of the surgical instrument, and a processor configured for controlling the actuator in accordance with the positioning commands.

It is common during intraocular surgical procedures to grasp retinal surface structures, such as retina membranes.

A surgical robotic system may assist a human operator in grasping a retinal surface structure. For example, the surgical instrument held by the surgical robotic system may be provided with a grasping device, such as a forceps or a micro vacuum pick. Using the surgical robotic system, and in particular when using the surgical robotic system in a master-slave configuration, the human operator may carry out the grasping action with enhanced precision, improved dexterity, steadier instrument manipulation, the ability to scale movements, etc., compared to a fully manual grasping action.

EP4104779A1 describes a surgical robotic system for ocular surgery, comprising a surgical arm with a movable arm part having at least one degree of freedom for longitudinal movement of a surgical instrument. A processor subsystem determines a displacement distance based on sensor data, outputs the distance via a user interface, and actuates the arm after receiving a confirmation signal.

US2022/265383A1 describes an imaging-based strategy for quantifying, tracking, and mitigating tractive forces on a retina during retinal surgery.

US9907696B2 describes a motion-compensated micro-manipulation system which uses optical coherence tomography with a motion-compensated micro-manipulation systems, including surgical blades.

US 2019/307523A1 describes a surgical probe system comprising a surgical probe having an instrument tip, and at least one motion sensor located within the surgical probe that measures movement and orientation data. The system further includes a processor that is configured to determine movement and orientation of the instrument tip based on the movement and orientation data, and adjust at least one surgical parameter of the surgical probe based on the movement and orientation of the instrument tip to affect a predetermined surgical outcome.

US 2021/228284A1 describes an eye surgery surgical system including a visualization device for displaying a relative position of a section of a surgery object in a 3D reconstruction of a region of a patient's eye.

### SUMMARY

Nevertheless, the inventors recognized that it may still be difficult for the human operator to estimate how deep to venture into the tissue of the retinal surface structure to actually grasp the retinal surface structure without damaging the retina. A surgeon often has to try several times to grasp a retinal surface structure, each time risking retinal damage. There is thus a high risk of retinal damage due to (mis)grasps, even when operating the grasping device using a surgical robotic system.

It would be desirable to obtain a surgical robotic system which is capable of further assisting with the task of grasping a retinal surface structure.

In a first aspect of the invention, a surgical robotic system as defined by claim 1 is provided for use in an intraocular procedure.

In a further aspect of the invention, a computer program as defined by claim 16 is provided, wherein the computer program comprises instructions, which when executed by a processor subsystem of a surgical robotic system, cause the processor subsystem to control the surgical robotic system during an intraocular procedure.

The above aspects of the invention provide a surgical robotic system which comprises a surgical arm. The surgical arm may comprise a movable arm part, and the movable arm part may comprise an end effector for holding a surgical instrument. For example, the end effector may comprise an instrument connector for mounting the surgical instrument, e.g., in a removable manner. The surgical instrument may typically have a longitudinal axis which may pass through a tip of the surgical instrument. The movable arm part may have at least one Degrees-of-Freedom (DoF) to move the tip of the surgical instrument longitudinally, for example to advance towards and/or retract away from a surgical target. The movable arm part may typically have three or more DoFs, for example to enable lateral movement of the tip of the surgical instrument along a plane perpendicular to the longitudinal axis. In general, the movable arm part may have sufficient DoFs enable the surgical instrument to be adjustable in pose, e.g., in position and orientation. It is noted that surgical arms having the functionality described in this paragraph are known per se from the field of medical robotics, and also known as instrument manipulators, robotic arms, surgical robot slave devices, etc.

An actuator subsystem may be provided for actuating the movable arm part to adjust the pose of the movable arm part, and thereby to adjust the pose of the surgical instrument. Another term for actuator subsystem is driving mechanism.

A processor subsystem may be provided for controlling the actuator subsystem to control the pose of the surgical instrument during the intraocular procedure. In some embodiments, the processor subsystem may be configured to receive positioning instructions from a human operator, for example via a user input interface, and control the actuator subsystem in accordance with the positioning instructions. Such type of control may also be referred to as master-slave control. It is noted that the processor subsystem may also be configured to control the pose of the surgical instrument semi-autonomously or even fully autonomously, for at least part of the intraocular procedure. During such (semi-)autonomous operations, there may be a possibility for the human operator to override an autonomous operation.

To enable the surgical robotic system to grasp a retinal surface structure, the surgical instrument may be further provided with a grasping device. Non-limiting examples of a grasping device include a forceps and a micro vacuum pick.

The surgical robotic system may be further provided with a sensor which is attached to or integrated into an intraocular part of the surgical instrument. The sensor may be used by the surgical robotic system to estimate a distance between the grasping device and a retinal surface. For example, the sensor may be an optical coherence tomography (OCT) probe which may be attached to or integrated into an intraocular part of the surgical instrument. The OCT probe may for example be, or comprise, an optical fibre which is connected to an OCT sensor, with the OCT sensor being located more distally, e.g., outside of the eye. The OCT probe may be forward facing, in that it may face along the longitudinal axis forward of the tip of the surgical instrument. In some embodiments, the OCT probe may additionally, or alternatively, be sideward facing. For example, the OCT probe may be a multi-directional probe with axial and side-viewing capability so as to be able to detect the presence of a feature of interest also sideward to the tip of the surgical instrument. Other examples of sensors include, but are not limited to, a stereo camera arranged for image capture through a microscope, an optical interferometric sensor integrated in or attached to the surgical instrument, a time-of-flight sensor integrated in or attached to the surgical instrument and an ultrasonic sensor integrated in or attached to the surgical instrument, etc.

Using the sensor, the processor subsystem may obtain sensor data which may allow the processor subsystem to estimate the distance to the retinal surface, and thereby the distance to the retinal surface structure. The processor subsystem may be configurable to operate in a grasping mode. This grasping mode, which may for example be activated by the human operator or automatically activated at a certain stage in the intraocular procedure, may assist in the grasping of the retinal surface structure. Namely, when the grasping mode is activated, the processor subsystem may control the actuator subsystem to adjust the pose of the surgical instrument to position the grasping device at a desired predetermined distance to the retinal surface structure. Typically, this may involve advancing the surgical instrument towards the retinal surface, for example by adjusting the position and/or orientation of the surgical instrument relative to the retinal surface. The predetermined distance may for example be a distance at which the retinal surface structure may be grasped, but without advancing too far towards the retinal surface to avoid damaging the retina.

After arriving at the desired position, the grasping device may be operated by the processor subsystem to grasp the retinal surface structure. For example, a forceps may be operated to close its arms, or a micro vacuum pump may be operated to start generating suction. It will be appreciated that in order to support such operation by the processor subsystem, the grasping device may be remotely actuatable by the surgical robotic system, e.g., via one or more actuators. Such actuator(s) may for example be part of the grasping device, the surgical instrument and/or the surgical arm.

The above measures may have the effect that during an intraocular procedure, the surgical robotic system may further assist with the task of grasping a retinal surface structure, with 'further' referring to going beyond the known improvements obtained by using a master-slave surgical robotic system such as improved dexterity, steadier instrument manipulation, the ability to scale movements, etc. Namely, the surgical robotic system may be provided with a purposefully designed grasping mode, which when activated, may carry out two of the primary phases of a grasping procedure automatically, namely a first phase in which the surgical instrument is adjusted in pose to reach a suitable position at which the retinal surface structure can be grasped by the grasping device, and in a second phase, the actual grasping action. When using such a grasping mode, the human operator may be relieved from his/her responsibility to estimate how deep to venture into the tissue of the retinal surface structure to actually grasp the retinal surface structure without damaging the retina. Rather, it has been found that this distance can be sufficiently reliably estimated by sensor(s), thereby enabling the positioning of the grasping device to be delegated to the surgical robotic system. This correct positioning in particular has been found to be difficult to achieve manually, as evidenced by surgeons often having to try several times to grasp a retinal surface structure, each time risking retinal damage. In addition, by also activating the grasping automatically, this phase is also delegated to the processor subsystem, which may enable the processor subsystem to control various aspects of this phase, for example the timing, to more safely and reliably carry out the grasping. Advantageously, by providing a purposefully designed grasping mode, there is a decreased risk of (mis)grasps, and thereby a decreased risk of retinal damage.

The following optional aspects are described with reference to the surgical robotic system but may equally apply to the computer-implemented method for controlling the surgical robotic system and to the corresponding computer program.

Optionally, the processor subsystem is configured to activate the grasping mode in response to a user command. The grasping mode may be a user-selectable mode. The human operator may therefore manually activate the grasping mode, for example via a user input interface. Such activation may for example take place when the human operator considers the grasping of the retinal surface structure to be a next step in the intraocular procedure, or when a grasping of the retinal surface structure through direct control of the human operator has previously failed. Alternatively, or additionally, the processor subsystem may automatically activate the grasping mode in certain situations, for example when the processor subsystem considers the grasping of the retinal surface structure to be a next step in the intraocular procedure
Optionally, the processor subsystem is configured to operate the grasping device in response to a user command. While the aforementioned first phase and the second phase of a grasping procedure may both be carried out automatically by the surgical robotic system, the second phase may, but does not need to, directly follow the first phase. For example, in some embodiments, the surgical robotic system may prompt the human operator for a confirmation to proceed before proceeding to grasp the retinal surface structure with the grasping device. To enable the human operator to make an informed decision, sensory perceptible feedback may be provided to the human operator, such as the current distance to the retinal surface structure, one or more characteristics of the retinal surface structure as obtained from sensor data, etc. Another example is that the surgical robotic system may verify, after positioning, whether such grasping is likely to be successful, e.g., based on sensor data obtained at the new instrument position, and may provide such feedback to the human operator to enable the human operator to confirm whether to proceed with the actual grasping.

Optionally, the processor subsystem is configured to generate and output sensory perceptible feedback which characterizes one or more operational parameters associated with the grasping mode. Such feedback may assist a human operator in making an informed decision on whether to, e.g., activate the grasping mode, or when having activated the grasping mode, continue with a first phase or second phase of the grasping mode, or deactivate the grasping mode or abort the grasping procedure, etc.

Optionally, the processor subsystem is configured to, after grasping the retinal surface structure with the grasping device, control the actuator subsystem to perform a post-grasp movement with the grasping device, for example a retreating movement. The intraocular procedure may, following the grasping of the retinal structure, prescribe a post-grasp movement, such as a movement towards or away from the retina, for example with a predetermined magnitude. There may be various motivations for such a post-grasp movement, for example to mechanically manipulate the retinal surface structure, e.g., to detach, tear or relocate parts of the retinal surface structure, to assess a quality of grasp of the retinal surface structure, to increase the distance between the grasping device and the retinal surface for safety reasons, etc. The processor subsystem may be configured to automatically perform the post-grasp movement, for example immediately following the actual grasping of the retinal surface structure. The post-grasp movement may for example be predetermined by the human operator, by a look-up table, by a calibration procedure, etc. In some examples, the post-grasp movement may be specific to the type of grasping device and/or surgical instrument, in which case the look-up table and the calibration procedure may also be type-specific. By also delegating the post-grasp movement to the processor subsystem, the processor subsystem may be enabled to control various aspects of this phase, for example the timing, to more safely and reliably carry out the post-grasp movement.

Optionally, the processor subsystem is configured to, before operating the grasping device to grasp the retinal surface structure, control the actuator subsystem to perform a pre-grasp movement with the grasping device. It may not only be relevant to position the grasping device at the predetermined distance to the retinal surface structure, but also that a pre-grasp movement is performed with the grasping device. There may be different motivations for such a pre-grasp movement. For example, via a pre-grasp movement, the orientation of the grasping device relative to the retinal surface structure may be adjusted, or a mechanical manipulation may be carried-out (e.g., a limited lateral movement to create a flap with a micro vacuum pick, or to lift-up the retina), etc. By also delegating the pre-grasp movement to the processor subsystem, the processor subsystem may control various aspects of this phase, for example the timing, to more safely and reliably carry out the pre-grasp movement.

Optionally, the pre-grasp movement comprises a rotation to adjust an orientation of the surgical instrument in relation to an orientation of the retinal surface structure, wherein the processor subsystem is configured to adjust the orientation of the surgical instrument by rotating the surgical instrument about a longitudinal axis of the surgical instrument. The success and/or the quality of the grasp which is achieved may not only depend on the grasping device having a suitable position relative to the retinal surface structure, but also on the grasping device having a suitable orientation relative to the retinal surface structure when attempting to grasp the retinal surface structure. By way of a pre-grasp movement, the grasping device may be reoriented to achieve this suitable orientation. Such reorientation may for example be performed before or while repositioning the grasping device to reach the desired distance to the retinal surface structure. Advantageously, by adjusting the orientation before reaching the desired distance, the chance of damage to the retinal surface may be reduced.

Optionally, the processor subsystem is configured to determine the orientation of the retinal surface structure using a geometric model of the retinal surface structure or of the retina, wherein the geometric model is parameterized, and wherein the processor subsystem is configured to determine one or more parameters of the geometric model based on the sensor data. The optimal or an at least suitable orientation of the grasping device may depend on the orientation of the retinal surface structure. To be able to determine the latter orientation, a geometric model of the retinal surface structure may be used, or a geometric model of the retina if the model of the retina is indicative of the orientation of the retinal surface structure. The geometric model may be parametrized and, via the adjustment of parameters, fitted to the actual retinal surface structure or retina at hand. To achieve the fitting of the geometric model, the processor subsystem may analyse the sensor data to determine the parameters of the geometric model. This way, the processor subsystem may take the orientation of the retinal surface structure into account when reorienting the grasping device.

Optionally, the processor subsystem is configured to determine the orientation of the surgical instrument and/or the orientation of the retinal surface structure based on at least one of:
- the sensor data obtained from the sensor;
- extraocular sensor data obtained from an extraocular sensor, such as an optical coherence tomography, OCT, sensor, or a surgical microscope; and
- preoperative data indicative of the orientation of the retinal surface structure.

Optionally, the grasping device comprises a forceps with arms, wherein the processor subsystem is configured to rotate the surgical instrument to position the arms of the forceps at respective predetermined distances to the retinal surface structure, for example by positioning the arms equidistantly to the retinal surface structure. The grasping device may be or may comprise a forceps. Such forceps typically comprise two or more arms, which may also be referred to as jaws. To be able to successfully grasp the retinal surface structure, it may be desirable to position the arms at predetermined distances to the retinal surface structure before proceeding with the actual grasping. For example, when the arms have the same length, e.g., in a symmetric forceps design, it may be desirable for each arm to have the same distance to the retinal surface structure when grasping the retinal surface structure. Another example is that when the arms do not have a same length (e.g., a jaw for executing a lifting motion and a jaw for executing the grasping), it may be desired to position the arms at different distances to the retinal surface structure before grasping.

Optionally, the grasping device comprises a micro vacuum pick, wherein the processor subsystem is configured to rotate the surgical instrument to align an aspiration port of the micro vacuum pick with the retinal surface structure. Similarly as in the case of forceps, it may also be desirable to (re)orient a micro vacuum pick to be able to successfully grasp the retinal surface structure. For example, it may be desirable to align the aspiration port towards the retinal surface structure.

Optionally, the processor subsystem is configured to determine a quality of the grasp of the retinal surface structure based on at least one of:
- the sensor data obtained from the sensor;
- further sensor data obtained from a further intraoperative sensor, such as an intraoperative impedance or admittance sensor; and
- extraocular sensor data obtained from an extraocular sensor, such as an optical coherence tomography, OCT, sensor, or a surgical microscope.

After grasping the retinal surface structure, the processor subsystem may determine the quality of grasp, referring to the assessment of factors such as the firmness of the grip, the degree of contact between the jaws and the retinal surface structure, and the stability of the hold during manipulation. To be able to assess the quality of grasp, sensor data may be used which may be obtained from different sources. The assessment of the quality of grasp may allow the processor subsystem to assess a success of the grasping action, which in turn may be used as feedback towards the human operator, to determine a next step in the intraocular procedure, etc.

Optionally, the processor subsystem is configured to trigger a remedial action when the quality of the grasp is determined not to meet a quality threshold. For example, the processor subsystem may be configured to generate and output sensory perceptible feedback to the user, e.g., as a warning, or to operate the grasping device of the surgical instrument to release and/or to regrasp the retinal surface structure.

Optionally, the sensor data is indicative of periodic physiological movement of a patient, and wherein the processor subsystem is configured to estimate the periodic physiological movement from the sensor data and account or compensate for the periodic physiological movement when controlling the actuator subsystem and/or operating the grasping device. Certain periodic physiological movement may have an effect on an intraocular procedure, for example the natural pulsation of the heart causing minute eye movements, respiratory cycles influencing ocular pressure, and involuntary micro saccades or eye twitching. In some situations, periodic physiological movement may pose a risk, for example when the grasping device is positioned very near the retinal surface in preparation for grasping the retinal surface structure. To be able to account or compensate for such periodic physiological movement, the processor subsystem may be configured to estimate the physiological movement from the sensor data, e.g., using signal filtering, pattern recognition, etc. This way, the risk posed by the periodic physiological movement may be reduced or avoided.

Optionally, the processor subsystem is configured for at least one of:
- control the actuator subsystem to adjust the pose of the surgical instrument to compensate for the periodic physiological movement;
- control the actuator subsystem to, when adjusting the pose of the surgical instrument, account for the periodic physiological movement; and
- control the actuator subsystem to operate the grasping device to grasp the retinal surface structure at a time when the periodic physiological movement is estimated to least affect said grasping.

The periodic physiological movement may affect the relative position and/or orientation between the grasping device and the retinal surface structure. By compensating for the periodic physiological movement, a current relative position and/or orientation may be maintained. By accounting for the periodic physiological movement, an anticipated change in relative position and/or orientation may be used pro-actively to achieve a desired effect. For example, when repositioning the grasping device, the repositioning may be aligned or timed with an anticipated physiological movement. Another example is that when the periodicity of the physiological movement is known, actions relating to the grasping procedure, such as the actual grasping, may be timed so that they are least affected by the physiological movement. This may avoid having to compensate for the physiological movement, which may not always be possible or which require the physiological movement to be known more precisely.

Optionally, the processor subsystem is configured to control the actuator subsystem to adjust the pose of the surgical instrument using one of:
- closed-loop control in which the sensor data obtained from the sensor is used during movement of the grasping device to update an initial estimate of the distance to the retinal surface structure;
- open-loop control in which the initial estimate is not updated during the movement of the grasping device.

Closed-loop control may provide the advantage that it may be possible to compensate for changes in the environment during the movement of the grasping device. For example, there may be external factors causing the distance to the retinal surface structure to be changed, e.g., due to a positioning of the patient or the aforementioned periodic physiological movement. By continuing to estimate the distance to the retinal surface structure during the movement, it may be avoided that the grasping device does not reach the predetermined distance or overshoots. An advantage of open-loop control may be the following. Sensor measurements carried out during movement may be noisy. By not using such measurements, the influence of measurement noise on the positioning of the grasping device may be reduced.

Optionally, the sensor is one or a combination of: an OCT probe, a force-based sensor, and an impedance or admittance sensor.

Modifications and variations of the computer-readable medium, which correspond to the described modifications, variations, and optional aspects of the surgical robotic system, may be carried out by a person skilled in the art on the basis of the present description.

### BRIEF DESCRIPTION OF THE DRAWINGS

These and other aspects of the invention are apparent from and will be elucidated with reference to the embodiments described hereinafter. In the drawings,
Fig. 1 shows a schematic representation of a surgical robotic system;
Fig. 2 shows a surgical instrument passing through a trocar during minimally invasive surgery, the surgical instrument having four degrees of freedom (DoFs);
Fig. 3 shows a joint diagram illustrating the kinematics of a movable arm part of a surgical arm for use in minimally invasive surgery;
Fig. 4 shows a joint diagram illustrating the kinematics of a motion controller;
Fig. 5 shows a surgical instrument in an eye according to an embodiment of the invention;
Fig. 6A shows the surgical instrument with its grasping device at an initial distance relative to a retinal surface structure;
Fig. 6B shows the surgical instrument with its grasping device having been advanced to reach a target distance with respect to the retinal surface structure;
Fig. 6C shows the grasping device having been operated to grasp the retinal surface structure;
Fig. 6D shows a post-grasp movement of the grasping device;
Figs. 7A-B show a pre-grasp movement of the grasping device in form of a rotation about its longitudinal axis to equidistantly position the arms of the grasping device relative to the retinal surface structure;
Fig. 8 schematically shows a method for controlling a surgical robotic system during an intraocular procedure; and
Fig. 9 shows a non-transitory computer-readable medium;

It should be noted that items which have the same reference numbers in different figures, have the same structural features and the same functions, or are the same signals. Where the function and/or structure of such an item has been explained, there is no necessity for repeated explanation thereof in the detailed description.

### List of reference numerals

The following list of references and abbreviations is provided for facilitating the interpretation of the drawings and shall not be construed as limiting the claims.
- **20**: **user interface** subsystem
- 22: user inputs
- 30: sensor
- 32: sensor data
- 40: processor subsystem
- 42: actuation commands
- 60: actuator subsystem
- 62: actuation of surgical arm
- 80: surgical arm
- 82: movable arm part
- 100: surgical robotic system
- 104: *e̅ₓ*, axis of coordinate system fixed to the instrument tip, orthogonal to the instrument longitudinal axis
- 105: *e̅_{y},* axis of coordinate system fixed to the instrument tip, orthogonal to the instrument longitudinal axis
- 106: *̅e̅*̅_{z}, axis of a cartesian coordinate system, aligned with the instrument longitudinal axis
- 107: *ϕ*, rotation of surgical instrument, laterally displacing its tip
- 108: *Ψ*, rotation of surgical instrument, laterally displacing its tip
- 109: z, longitudinal (along its longitudinal axis) translation of surgical instrument, or penetration direction, or advancing direction
- 110: θ, rotation of surgical instrument around its longitudinal axis
- 111: *φ*, rotational DoF of a movable arm part
- 112: *ψ*, rotational DoF of a movable arm part
- 113: *z*, translational DoF of a movable arm part
- 114: *θ*, rotational DoF of a movable arm part
- 115: *φₘ,* rotational DoF of motion controller
- 116: *ψₘ*, rotational DoF of motion controller
- 117: *zₘ*, translational DoF of motion controller
- 118: *θₘ*, rotational DoF of motion controller
- 119: surgical instrument
- 121: sensor or sensor part
- 122: grasping device
- 123: surgical target
- 124: trocar
- 125: remote centre of motion (RCM)
- 126: button on motion controller gripper
- 127: optical beam
- 200: eye
- 210: retina
- 220: first layer of tissue
- 230: second layer of tissue
- 240: retinal surface structure
- 250: audio signal
- 252: silenced audio signal
- 300: positioning grasping device relative to retinal surface structure
- 310: actuation of grasping mechanism
- 320: post-grasp movement
- 330: pre-grasp movement
- 400: method of controlling a surgical robotic system
- 410: controlling actuator subsystem
- 420: activating grasping mode
- 430: using actuator subsystem, positioning grasping device at predetermined distance to retinal surface structure
- 440: is predetermined distance reached?
- 450: operating grasping device to grasp retinal surface structure
- 500: non-transitory computer readable medium
- 510: data representing computer program

### DESCRIPTION OF EMBODIMENTS

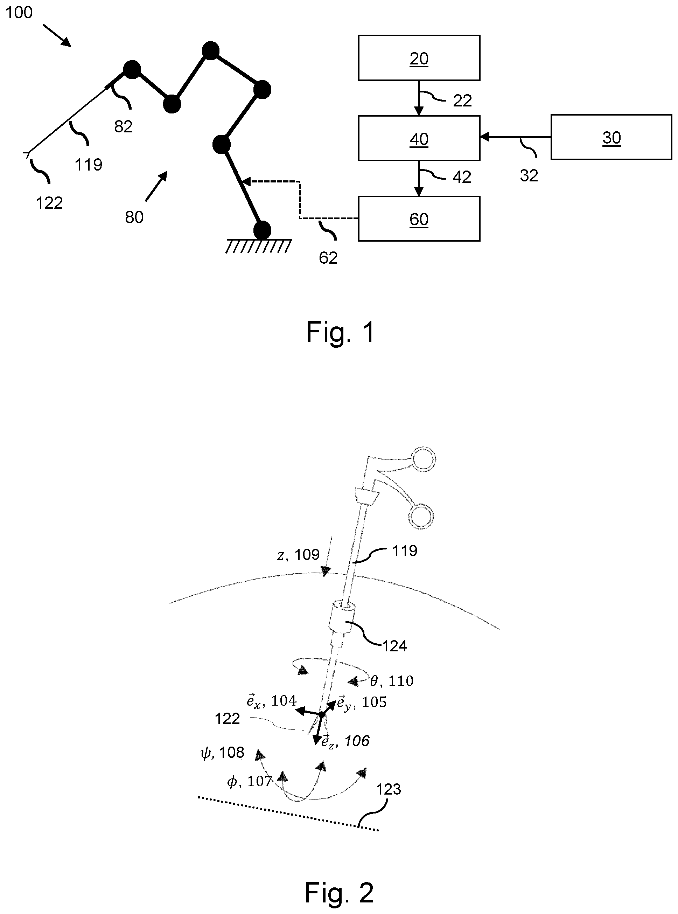

The following embodiments relate to a surgical robotic system for use in an intraocular procedure. The surgical robotic system may comprise a movable arm part. The movable arm part may comprise an end effector for holding a surgical instrument. The surgical instrument may comprise a grasping device for grasping a retinal surface structure. The surgical robotic system may further comprise an actuator subsystem configured to actuate the movable arm part. During the intraocular procedure, the surgical robotic system may, when a grasping mode is activated, control the actuator subsystem to adjust the pose of the surgical instrument to position the grasping device at a predetermined distance to a retinal surface structure, and after reaching the predetermined distance to the retinal surface structure, operate the grasping device of the surgical instrument to grasp the retinal surface structure. The following describes the grasping mode and its functionality within the context of a specific surgical robotic system, which is described with reference to, inter alia, Figs. 1-4. Nonetheless, the embodiments described in this specification are not confined solely to this type of surgical robotic system. Rather, the embodiments can be adapted and applied to other types of surgical robotic systems for use in intraocular procedures.

Fig. 1 schematically shows a surgical robotic system 100 for use in an intraocular surgical procedure. The surgical robotic system 100 may comprise a surgical arm 80. The surgical arm 80 may comprise a movable arm part 82 which may comprise an end effector (not explicitly shown in Fig. 1) for holding a surgical instrument 119. To hold the surgical instrument 119, the end effector may comprise an instrument connector to which the surgical instrument 119 may be mounted.

The surgical instrument 119 may comprise a grasping device 122, such as a forceps or a micro vacuum pick. In some embodiments, the surgical instrument 119 may in its entirely be considered a grasping device. In other words, the surgical instrument 119 may be the grasping device. In other embodiments, the grasping device 122 may be separate from the surgical instrument 119, for example by being mounted removably or non-removably to the surgical instrument. In such embodiments, the grasping device 122 may be held by the surgical instrument 119, for example at its distal end, for example at or near its tip. In some embodiments, the surgical instrument 119 may comprise a grasping device and one or more other intraoperative devices. In general, the grasping device 122 may be actuatable to perform a grasping action. The actuation may be mechanical, electrical, pneumatically, or otherwise. In embodiments where the grasping device is separate from, but held by, the surgical instrument 119, the actuation of the grasping device may take place via the surgical instrument.

The movable arm part 82 may have at least three degrees-of-freedom (DoF), and preferably at least six DoFs, to enable adjustment of the pose of the surgical instrument, and thereby adjustment of the pose of the grasping device. For example, the movable arm part 82 may have DoFs to enable longitudinal movement of the surgical instrument towards a surgical target within the eye and lateral movement of the surgical instrument in a plane normal to said longitudinal movement. Here, longitudinal movement may refer to a movement of the surgical instrument 119 along its longitudinal axis (not separately shown in Fig. 1). In some embodiment, the surgical robotic system 100 may be configured to enable lateral movement in a curved plane. The curvature of the curved plane may for example follow a curvature of the interior of the eye. Such type of lateral movement along a curved plane may be obtained by the surgical robotic system adjusting the longitudinal position of the surgical instrument 119 during lateral movement. The shape of the curved plane may for example be determined based on a model of the eye or an intraocular structure, e.g., the retina.

In some embodiments, the surgical robotic system 100 may further comprise a user interface subsystem 20 for receiving user inputs 22 from a user. The user interface subsystem 20 may thus represent at least a user *input* interface, and in some embodiments, as also elucidated elsewhere, additionally or alternatively a user *output* interface. The user inputs 22 may for example comprise positioning instructions from a human operator, e.g., a surgeon, to enable the human operator to determine a trajectory of the surgical instrument. In some embodiments, the positioning instructions may be positioning commands for directly controlling the movement of the surgical instrument. In other embodiments, the positioning instructions may be provided as input during a planning procedure in which the trajectory of the surgical instrument is planned. Other types of user inputs may for example include confirmation inputs indicating instructions to continue with a procedure or procedural step, and/or input information, such as indicating an operating parameter and/or an indication of a surgical target position or the like. Examples of user input interfaces for receiving user inputs from a user include, but are not limited to, a keyboard, a mouse, a touchsensitive surface, a joystick, a foot pedal, a microphone, a gesture recognition system, etc. The user input interface may employ any suitable input modality, such as touch, push-actions, voice commands, eye movements, gesture recognition, etc.

The surgical robotic system 100 may further comprise an actuator subsystem 60 configured and arranged for actuating the movable arm part to effect the longitudinal movement and lateral movement of the surgical instrument. The actuator subsystem 60 may comprise any suitable actuator(s), e.g., from the field of surgical robots, or from the more general field of actuators. In particular, the actuator subsystem 60 may comprise a plurality of actuators which together provide the actuation of the movable arm part 60 along the three or more DoFs. Accordingly, it will be appreciated that any reference to a specific configuration of the actuator subsystem 60 may be understood as referring to a (joint) configuration of such a plurality of actuators.

Fig. 1 shows the actuation of surgical arm 80 schematically, namely as a dashed line 62. It is noted that, although shown separately of the surgical arm 80, the actuator subsystem 60 may be integrated into, or mounted to, the surgical arm 80.

The surgical robotic system 100 may further comprise a sensor 30 which may be configured to generate sensor data which is indicative of a distance between the grasping device and a retinal surface. For example, the sensor 30 may be or comprise an optical coherence tomography (OCT) probe. The OCT probe may for example be an optical fibre which is attached to or integrated in the surgical instrument 119, with the optical fibre being connected to a remotely located OCT sensor. In other examples, the OCT probe may comprise an OCT sensor which is directly attached to or directly integrated in the surgical instrument 119. The sensor data 32 provided by the OCT probe may comprise A-scans, which may comprise line measurements and defined as an intensity as a function of the distance, B-scans forming a 2D image, C-scans, e.g., from multiple B-scans, or the like. It is noted that even though the sensor 30 is shown in Fig. 1 to be separate from the surgical instrument 119, the sensor or part of the sensor may be attached to or integrated in the surgical instrument 119. In other examples, the sensor 30 may, in addition or alternatively to the OCT probe, comprise a stereo camera arranged for image capture through a microscope, an optical interferometric sensor integrated in or attached to the surgical instrument 119, a time-of-flight sensor integrated in or attached to the surgical instrument and an ultrasonic sensor integrated in or attached to the surgical instrument, a force-based sensor integrated in or attached to the surgical instrument, or an impedance or admittance sensor integrated in or attached to the surgical instrument. Although not shown in Fig. 1, in yet other examples, the surgical robotic system may comprise one or more additional sensors, such as an extraocular sensor, e.g., an extraocular OCT sensor, or an additional intraocular sensor, e.g., an intraoperative impedance or admittance sensor. In some embodiments, the surgical robotic system 100 may comprise a microscope which may be communicatively coupled to the surgical robotic system 100.

The surgical robotic system 100 may further comprise a processor subsystem 40 configured for controlling the actuator subsystem 60 to control a pose of the surgical instrument during the intraocular procedure. For the purpose of controlling the actuator subsystem 60, the processor subsystem 40 may provide actuation commands 42 to the actuator subsystem. Thereby, the processor subsystem 40 may adjust a position and orientation of the grasping device during the intraocular procedure. As also elucidated elsewhere, the processor subsystem 40 may function in an operator control mode in which the processor subsystem 40 directly carries out positioning commands received from a human operator, or in an autonomous control mode in which the processor subsystem 40 autonomously controls the position of the surgical instrument, e.g., according to a pre-planned trajectory and sensor data, or in a semi-autonomous control mode which combines aspects of the operator control mode and the autonomous control mode. Such a semi-autonomous control mode may for example comprise parts of the intraocular procedure being carried-out under direct control of the human operator, e.g., 'manually', while other parts of the intraocular procedure may be carried out without such direct control.

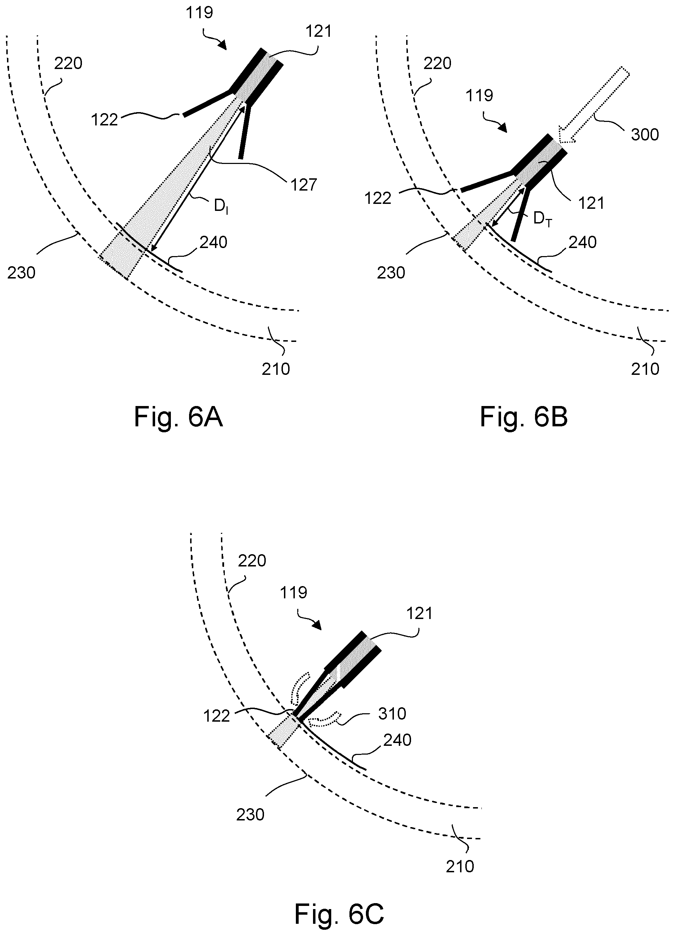

The processor subsystem 40 may be configured to operate in a grasping mode. The grasping mode may be activatable by the human operator, for example via the aforementioned user interface subsystem 20. Alternatively, or additionally, the grasping mode may be activatable by the processor subsystem 40 itself, for example at a certain phase of the intraocular procedure. The processor subsystem 40 may be configured to, when the grasping mode is activated, carry out certain actions pertaining to the grasping of the retinal surface structure. The different actions may elsewhere also be referred to as 'phases' or 'procedural steps' of the grasping. In particular, the processor subsystem 40 may control the actuator subsystem to adjust the pose of the surgical instrument to position the grasping device at a predetermined distance to a retinal surface structure, and after reaching the predetermined distance to the retinal surface structure, operate the grasping device of the surgical instrument to grasp the retinal surface structure. These and other of the operation of the surgical robotic system 100 will be further elucidated with reference to, inter alia, Figs. 6A-7B.

While the positioning of the grasping device and the operating of the grasping device, e.g., the actual grasping, may be carried out autonomously by the processor subsystem, the processor subsystem 40 may be configured to involve the human operator. For example, the processor subsystem 40 may be configured to activate the grasping mode and/or operate the grasping device in response to a user command. For example, the processor subsystem 40 may, via the user input interface 20, prompt the human operator whether to proceed with each of both of said actions, and only proceed if a confirmatory user command is received. To enable the human operator to make an informed decision whether to proceed, and/or to generally keep the human operator informed, the processor subsystem 40 may be configured generate and output sensory perceptible feedback which characterizes one or more operational parameters associated with the grasping mode. For example, via the sensory perceptible feedback signal, the processor subsystem 40 may indicate a current distance between the grasping device and the retinal surface structure to the human operator, a current quality of grasp, etc. The sensory perceptible feedback signal may for example be an audible signal, a haptic signal, and/or a visual signal. For example, the processor subsystem 40 may be configured to output the sensory perceptible feedback signal via the user interface subsystem 20. In such an example, the user interface subsystem 20 may thus represent an output interface. For example, the sensory perceptible feedback signal may be rendered visually, e.g., as an overlay over an image of the surgical field, as an audio output, e.g., via a speaker of the user interface subsystem 20, and/or as a visual numerical output, e.g., on a display of the user interface subsystem 20. Another example is that a haptic signal may be provided via the user input interface, e.g., by providing a haptic signal via a motion controller.

**Fig.** 2 shows a surgical instrument 119 passing through a trocar 124 during minimally invasive surgery. For example, in case of vitreoretinal surgery, the trocar 124 may be placed in the sclera. Rotating around and translating through the trocar may be possible in four DoF, e.g., the rotations *ϕ* 107, *ψ* 108, *θ* 110 and the translation z 109 to approach or penetrate a surgical target 123. Further shown are a tip 122 of the surgical instrument 119 and three axes 104-106 of a coordinate system fixed to the instrument tip 122, with *e̅_{z}* 106 aligned with the longitudinal axis of the surgical instrument 119. Rotations *ϕ* 107 and *ψ* 108 may result in a lateral displacement of the instrument tip 122, respectively in the direction *e̅_{y}* 105 and in the direction *e̅ₓ* 104. The translation z 109 may result in a longitudinal movement of the surgical instrument tip 122.

The surgical robotic system 100 may be used in an intraocular surgical procedure, such as a minimally invasive surgical procedure as described above.

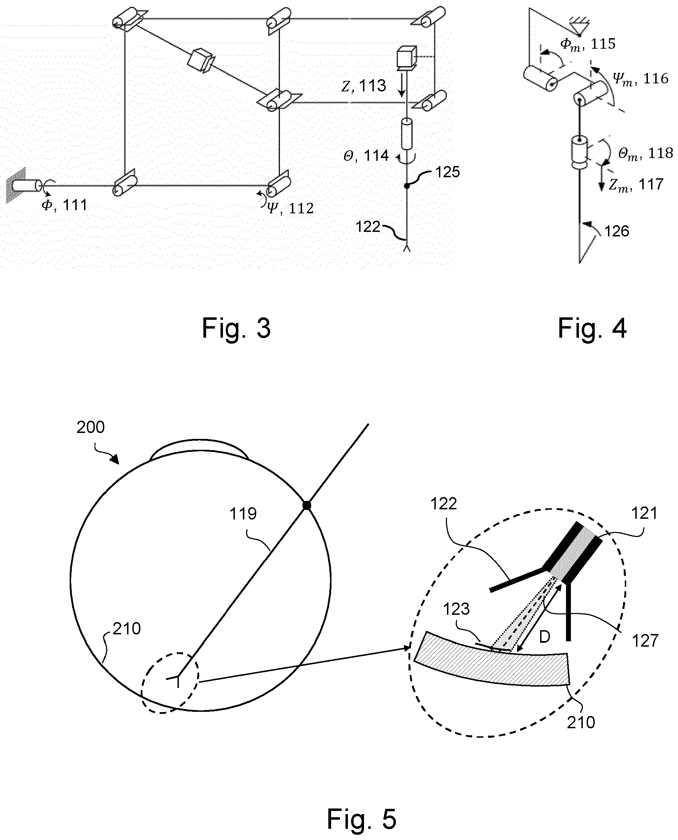

**Fig. 3** shows a joint diagram illustrating the kinematics of a movable arm part of a surgical arm for use in an intraocular surgical procedure, and in particular a minimally invasive surgery. In the example of Fig. 3, the surgical robotic system comprises a surgical arm, with the surgical arm comprising a movable arm part having DoFs *Φ* 111, *Ψ* 112, Z 113 and *Θ* 114, allowing instrument motions 107-110 as previously shown in Fig. 2, resulting in movements of the surgical instrument tip 122. The DoFs may be arranged such that there is a point on the surgical instrument 122 that does not move in space, termed the remote centre of motion (RCM) 125. By moving the base of the surgical arm, the movable arm part may be positioned such that the remote centre of motion 125 of the surgical instrument is positioned at the trocar. Respective actuators may be arranged to effect movement in all four DoFs 111-114.

As previously elucidated with reference to Fig. 1, the surgical robotic system may comprise a user input interface for receiving user inputs, such as positioning instructions, from a human operator, e.g., a surgeon or healthcare provider. In some embodiments, the user interface may comprise or be constituted by a motion controller such as a joystick. In some embodiments, the motion controller may be an external device with which the user interface or the processor subsystem 40 is configured to interface. In some embodiments, the motion controller may be comprised in the surgical robotic system as a further component, e.g., configured to interface with the processor subsystem 40 and/or the user interface subsystem 20. **Fig. 4** shows a joint diagram illustrating the kinematics of such a motion controller. Here, the motion controller is shown to have DoFs *Φₘ* 115, *Ψₘ* 116, *Zₘ* 117 and *Θₘ* 118. The user may provide user inputs, such as positioning commands to directly control the movement of the movable arm part, for example by holding the motion controller at a gripper part, pressing the button 126 and moving the gripper part of the motion controller in space.

**Fig. 5** shows a surgical instrument 119 in an eye 200. The tip of the surgical instrument 119 is also shown magnified. The tip of the surgical instrument 119 may be an operative tip in that the tip may be operatively involved in the intraocular procedure. In particular, as shown in Fig. 5, the tip of the surgical instrument 119 may be or comprise a grasping device 122, being in this example a forceps comprising a pair of arms. As illustrated on the left hand side of Fig. 5, the surgical instrument 119 is shown inserted into the eye 200. The surgical instrument 119 may be approaching the retina 210 as illustrated in this figure, although this is merely exemplary and the invention is not limited thereto. In some embodiments, the surgical instrument 119 may approach another area, structure, or tissue layer of the eye, such as a capsular bag or a part of the eye's drainage system. The grasping device 122 of the surgical instrument 119 is illustrated on the right hand side of Fig. 5, magnified. In this example, a surgical target 123 in form of a retinal surface structure is located on the surface of the retina. A sensor or sensor component 121, such as an optical fibre, may be integrated in or attached to the surgical instrument 119. In some embodiments, the sensor 30 may comprise an optical fibre (as shown) coupled to an OCT sensor and configured to emit an optical beam 127 to capture cross-sectional images of the retina 210 and other structures within the eye. The optical fibre 121 may be recessed relative to the arms of the grasping device 122. Since the OCT probe may be longitudinally forward facing, the sensor data may be indicative of the longitudinal distance D to the retinal surface structure laterally forward of the grasping device 122, which may be considered to be 'beneath' or 'ahead' of the grasping device 122. It will be appreciated that while the distance D may be larger than the distance of the distal tips of the grasping device 122 to the retinal surface structure, and thus may be considered to underestimate the proximity of the grasping device to the retinal surface structure, the dimensions of the grasping device 122 and its relative position to the optical fibre 121 or other sensor are typically known, which may allow the processor subsystem to determine the distance between the retinal surface structure to any part of the grasping device 122.

It is noted that when the surgical instrument 119 is operated in the periphery of the eye, or in the vicinity of the posterior side of the lens in vitrectomy, the retina and/or the retinal surface structure may also be sideways proximate to the grasping device, and in some cases more proximate sideways than longitudinally. To account for such sideward proximity, the OCT probe may be a multi-directional probe with axial and side-viewing capability so as to be able to determine the distance to the retinal surface structure and/or retina also sideward to the grasping device 122.

**Fig. 6A** shows the surgical instrument 119 of Fig. 5 with its grasping device 122 positioned at an initial distance Dᵢ relative to a retinal surface structure 240 which is located on or within a retinal surface (with the retina 210 being shown in Fig. 6A and elsewhere as an upper layer 220 and lower layer 230). Such an initial position may for example be obtained by the human operator having directly controlled the position of the surgical instrument 119, or as a result of the surgical robotic system having autonomously followed a certain trajectory. The initial distance Dᵢ may be too large to grasp the retinal surface structure 240, but may be large enough to reduce the risk of damaging the retinal surface to an acceptable degree. Such risk may otherwise be significant when the surgical instrument 119 is very proximate to the retina, e.g., due to the risk of inadvertent positioning errors of the surgical instrument 119 or external movement (e.g., due to periodic physiological movement). In the situation shown in Fig. 6A, as well as in various other situations, the grasping mode of the processor subsystem may be activated. **Fig. 6B** shows a result of the activation of the grasping mode. Namely, the processor subsystem adjusted the pose of the surgical instrument 119 so that the grasping device 122 is now positioned at a target distance D_{T} to the retinal surface structure 240. The target distance D_{T} may a distance at which the retinal surface structure can be grasped. For example, the target distance D_{T} may be predetermined, for example specifically for the type of grasping device 122 and/or the type of retinal surface structure. In other examples, the target distance D_{T} may be determined by the human operator, for example as a user-selectable parameter, etc. The pose of the surgical instrument may be adjusted by the processor subsystem using closed-loop control in which the sensor data obtained from the sensor is used during movement of the grasping device to update the initial estimate of the distance to the retinal surface structure. Alternatively, the pose of the surgical instrument may be adjusted by the processor subsystem using open-loop control in which the initial estimate is not updated during the movement of the grasping device. The processor subsystem may be configured to support either closed-loop control or open-loop control or both. In the latter case, the type of control may be selectable, e.g., by the human operator or depending on other factors, such as the type of sensor used.

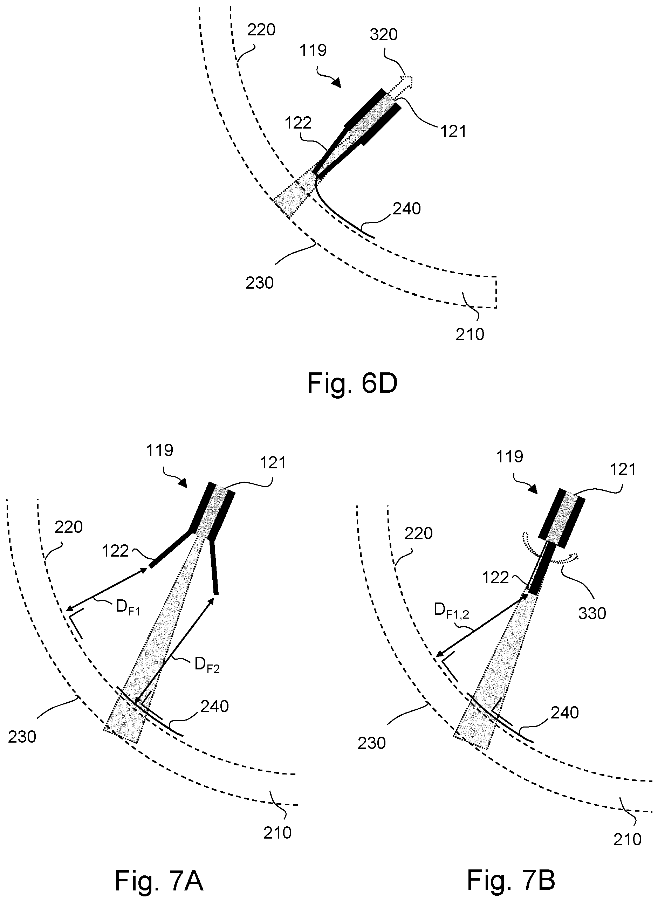

It is noted that while the adjustment of the pose of the surgical instrument 119 may result in a repositioning 300 of the grasping device 122, the adjustment of the pose may also effect a change in orientation of the grasping device 122. Such change in orientation will also be further elucidated with reference to Figs. 7A and 7B.

**Fig. 6C** shows the processor subsystem effecting a grasping action 310 with the grasping device 122 following the repositioning 300 of the grasping device previously shown in Fig. 6B. In some embodiments, such a grasping action 310 may directly and unconditionally follow the repositioning 300 of the grasping device. In other embodiments, the grasping action 310 may be performed only conditionally, for example subject to receiving a confirmation from the human operator or subject to analysis of sensor data obtained at the new position confirming that the grasping device 122 is in a suitable position for grasping the retinal surface structure. As may be known per se, the grasping action 310 may be carried out by actuating the grasping device 122, for example by actuating the arms of a forceps to mutually close, or by creating suction at an aspiration port of a micro vacuum pick, etc.

In some embodiments, the processor subsystem may be configured to, after or during the grasping action, determine a quality of the grasp of the retinal surface structure 240. For that purpose, the processor subsystem may access and analyse sensor data which may be indicative of the quality of grasp. For example, in some embodiments, the sensor data obtained from the sensor used for the distance measurements may be indicative of the quality of grasp. For example, the sensor may have imaging capabilities which may allow determining whether, and if so, in which way (e.g., to which extent, which part, etc) the retinal surface structure is grasped. Another example is that another intraoperative sensor may be used to assess the quality of grasp, such as an intraoperative impedance or admittance sensor. For example, an impedance or admittance sensor may be used to measure the impedance or admittance between the arms of a forceps to determine whether tissue was grasped by the forceps. As such, with sensing modalities such as impedance sensing at the grasping device or using an instrument-integrated OCT, the processor subsystem may measure if there is tissue connected to the grasping device, and if not, determine that a mis-grasp occurred. Yet another example is extraocular sensor data obtained from an extraocular sensor, such as an optical coherence tomography, OCT, sensor, or a surgical microscope. For example, such extraocular sensors may have imaging capabilities which may allow determining whether, and if so, in which way the retinal surface structure is grasped. More specifically, with such external sensing modalities, the tissue's reaction may be analysed while retracting. If there is tissue movement, the grasp may be deemed successful, and otherwise, a mis-grasp may have occurred.

In some embodiments, the processor subsystem may be configured to trigger a remedial action when the quality of the grasp is determined not to meet a quality threshold. The remedial action may for example comprise the processor subsystem generating and outputting sensory perceptible feedback to the user. Another example of a remedial action may be the processor subsystem operating the grasping device of the surgical instrument to release the retinal surface structure. Another example of a remedial action may be the processor subsystem operating the grasping device of the surgical instrument to regrasp the retinal surface structure, optionally after having again repositioned and/or reoriented the grasping device.

**Fig. 6D** shows a post-grasp movement 320 of the grasping device. The processor subsystem may be configured to control the actuator subsystem to perform such a post-grasp movement with the grasping device after grasping the retinal surface structure with the grasping device. While Fig. 6D shows the post-grasp movement to involve a repositioning 320, the post-grasp movement may also involve reorientation of the grasping device or a combination of a repositioning and reorientation. Fig. 6D shows the post-grasp movement to be a retreating movement 320 in which the surgical instrument 119 is moved longitudinally away from the retinal surface 220. Thereby, the retinal surface structure 240 may be pulled away from the surface of the retina 210, for example to prepare or start a peeling movement to remove the retinal surface structure. Another example of a post-grasp movement 320 may be a lateral (e.g., sideward) movement of the grasping device 122. The post-grasp movement may be predetermined, for example in form of a displacement vector or a trajectory. In some embodiments, data defining the post-grasp movement may be retrieved from, e.g., a look-up table, for example on the basis of the type of grasping device and/or the type of intraocular procedure. In other embodiments, the processor subsystem may be preconfigured with such data, thereby avoiding the need for a look-up table.

**Figs. 7A-B** show a pre-grasp movement 330 of the grasping device. The processor subsystem may be configured to control the actuator subsystem to perform such a pre-grasp movement with the grasping device before operating the grasping device to grasp the retinal surface structure. Figs. 7A-B show the pre-grasp movement to be a rotation 330 about the surgical tool's longitudinal axis. The purpose of such a rotation may be the following: it may be desirable to adjust the orientation of the surgical instrument in relation to the orientation of the retinal surface structure and/or the retina by rotating the surgical instrument about a longitudinal axis of the surgical instrument. For example, when the grasping device 122 is or comprises a forceps with a pair of arms, as in the example of Figs. 7A-B, to avoid damaging the retinal surface 220 with one of the arms, it may be preferred to have both arms positioned equidistantly to the retinal surface 220 when grasping the retinal surface structure 240. This may be achieved by the aforementioned rotation 330. In other words, the rotation 330 may cause the respective arms, which previously had different distances D_{F1}, D_{F2} to the retinal surface 220, to have a same or similar distance D_{F1, 2}. It is noted that Figs. 7A-B shows the distances D_{F1}, D_{F2}, D_{F1, 2} to be orthogonal to the retinal surface 220. This, however, is not a limitation, in that the distance may also be defined differently, e.g., longitudinally along the longitudinal axis of the surgical instrument 119.

While Figs. 7A-B illustrate a pre-grasp movement for a forceps, there may in general be different pre-grasp movements for different grasping devices and/or for different intraocular procedures. For example, if the grasping device is or comprises a micro vacuum pick, the processor subsystem may be configured to rotate the surgical instrument to align an aspiration port of the micro vacuum pick with the retinal surface structure. Moreover, while Figs. 7A-B show the pre-grasp movement to involve a reorientation, and specifically a rotation about the surgical tool's longitudinal axis, the pre-grasp movement may also involve any other type of reorientation of the grasping device or a repositioning or a combination of a repositioning and reorientation. It is further noted that the pre-grasp movement, and in particular a reorientation, may take place before positioning the grasping device at the desired target distance (e.g., D_{T} as shown in Fig. 6B) to the retinal surface structure to avoid damaging the retinal surface.

With continued reference to the reorientation, it is noted that the processor subsystem may be configured to determine the orientation of the grasping device relative to the orientation of the retinal surface structure in various ways. For example, their relative orientation may be directly determined from sensor data, e.g., from intraocular or extraocular sensor data as described elsewhere in this specification. In other examples, a geometric model of the retinal surface structure or of the retina may be used. The geometric model may be parameterized, for example in terms of size and shape (e.g., curvature). The processor subsystem may be configured to determine one or more parameters of the geometric model based on sensor data acquired during the intraocular procedure. This way, a patient-adapted model of the retina or retinal surface structure may be obtained, which may allow the processor subsystem to determine the orientation of the surgical instrument in relation to that of the retinal surface structure. In some embodiments, the sensor data obtained from the sensor used for the distance measurements may be used to determine the parameter(s) for the geometric model. For example, distance measurements at different positions within the eye may allow reconstruction of the curvature of the retinal surface structure. Additionally, or alternatively, extraocular sensor data from an extraocular sensor may be used, such as an optical coherence tomography, OCT, sensor, or a surgical microscope. Additionally, or alternatively, preoperative data may be used, e.g., obtained from a planning phase.

With continued reference to the reorientation of the grasping device, the following is noted. When no external depth sensing modality (e.g., external OCT) is used, local information of the retina may be used to determine the optimal tool orientation. In particular, the shape of the retina may be used at the intended grasping point. This shape may be determined from the aforementioned geometric model of the retina, or alternatively, or additionally, using test functions with which the retina shape may be determined at the intended grasping point using sensor data obtained from one or more intraoperative sensors. Such test functions may for example involve the surgical tool performing a sensor sweep by moving along a trajectory, such as a circle, a cross-like trajectory, etc. In such a sensor sweep, sensor data indicative of the local environment may be acquired by the intraoperative sensor, including but not limited to the local slope and local curvature profile of the retina. Another example is that the local layer structure of tissue underneath or ahead of the surgical instrument may be identified from the intraoperative sensor data, for example to identify vessels, or small gaps in the layer stack, etc. Yet another example of a test function may involve the surgical tool applying small forces to tissue underneath or ahead of the surgical instrument, for example using the vacuum generated by a micro vacuum pick. In a specific example, a small suction pulse may be applied at different lateral positions and the response of the tissue, e.g., the amount of displacement) may be observed from the sensor data. In general, when applying forces to the tissue, the response of the tissue may be observed using an intraocular sensor and/or an extraocular sensor. Test points may be laterally distributed according to a test pattern, such as the aforementioned circle or cross-like shape, or according to a line or 2D array.

In some embodiments, the processor subsystem may be configured with a geometry of the grasping device, and the geometry of the grasping device may be taken into account when reorienting the grasping device. For example, the geometry may indicate a length of the forceps' arms. When an external depth sensing modality is available, e.g., in form of the aforementioned extraocular OCT, the orientation of the grasping device may be controlled directly, for example by using the device-retina distances of different parts of the grasping device as feedback in a control loop by which grasping device is rotated. For example, for a forceps, the forceps may be rotated until the tips of both forceps' arms have the same or similar distance to the retina at the intended grasping point.

In some embodiments, the sensor data may be indicative of periodic physiological movement of a patient, and the processor subsystem may be configured to estimate the periodic physiological movement from the sensor data and account or compensate for the periodic physiological movement when controlling the actuator subsystem and/or operating the grasping device. For example, periodic physiological movements such as the natural pulsation of the heart causing minute eye movements, respiratory cycles influencing ocular pressure, and/or involuntary micro saccades or eye twitching may be detected by analysing the sensor data. For that purpose, signal analysis techniques such as Fourier transform analysis, wavelet transforms, autocorrelation analysis, real-time adaptive filtering, time-series analysis, etc, may be used to detect the periodic physiological movement from the sensor data. Another example is that a machine-learning based technique may be used. Having detected the periodic physiological movement, e.g., in terms of periodicity and optionally in terms of magnitude, future physiological movement may be predicted, e.g., by extrapolation according to the periodicity of the detected movement. This may allow the processor subsystem to control the actuator subsystem to adjust the pose of the surgical instrument to compensate for the periodic physiological movement. Additionally, or alternatively, the actuator subsystem may be controlled to, when adjusting the pose of the surgical instrument, account for the periodic physiological movement. For example, the size of the repositioning 300 shown in Fig. 6B may be adjusted based on an anticipated physiological movement which is expected to affect the distance D_{I} shown in Fig. 6A. Additionally, or alternatively, the actuator subsystem may be controlled to operate the grasping device to grasp the retinal surface structure at a time when the periodic physiological movement is estimated to least affect said grasping.

In general, the processor subsystem described in this specification may comprise one or more (micro)processors which execute appropriate software. The software implementing the functionality of the processor subsystem may have been downloaded and/or stored in a corresponding memory or memories, e.g., in volatile memory such as RAM or in non-volatile memory such as Flash. Alternatively, the processor subsystem may be implemented in the form of programmable logic, e.g., as a Field-Programmable Gate Array (FPGA). Any input and/or output interfaces may be implemented by respective hardware and/or software interfaces. In general, the processor subsystem, input interfaces, and/or output interfaces may each be implemented in the form of a circuit or circuitry. The processor subsystem may also be implemented in a distributed manner, e.g., involving different devices or apparatus.

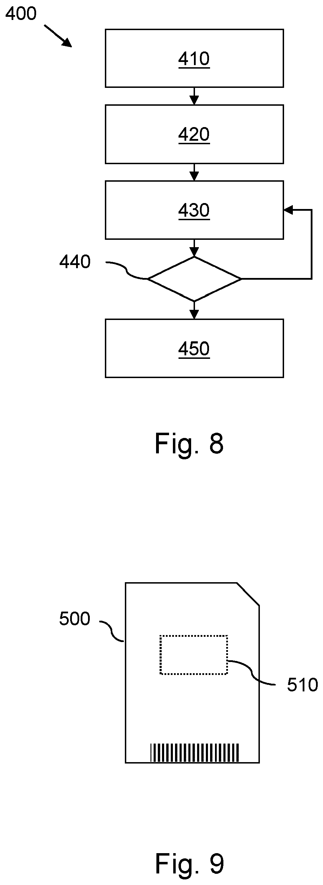

**Fig. 8** shows a method 400 of controlling a surgical robotic system during an intraocular procedure. The method 400 may comprise controlling 410 an actuator subsystem to control a pose of a surgical instrument during an intraocular procedure. The method 400 may further comprise, when a grasping mode is activated, controlling 430 the actuator subsystem to adjust the pose of the surgical instrument to position the grasping device at a predetermined distance to the retinal surface structure, and after reaching the predetermined distance to the retinal surface structure, operating 450 the grasping device of the surgical instrument to grasp the retinal surface structure.

It is further noted that, as shown in Fig. 8, the activation 420 of the grasping mode may be an integral step of the method 400, but may also be triggered externally causing the method 400 to proceed from step 410 to step 430. Moreover, as also shown in Fig. 8, the reaching of the predetermined distance may be verified in a step 440 in the method 440, but may also be may also be triggered externally, or simply assumed, causing the method 400 to proceed from step 430 to step 450.

It is noted that any of the methods described in this specification, for example in any of the claims, may be implemented on a computer as a computer implemented method, as dedicated hardware, or as a combination of both. Instructions for the computer, e.g., executable code, may be stored on a computer-readable medium 500 as for example shown in **Fig. 9****,** e.g., in the form of a series 510 of machine-readable physical marks and/or as a series of elements having different electrical, e.g., magnetic, or optical properties or values. The executable code may be stored in a transitory or non-transitory manner. Examples of computer-readable mediums include memory devices, optical storage devices, integrated circuits, servers, online software, etc. Fig. 9 shows by way of example a memory card 500.

It should be noted that the above-mentioned embodiments illustrate rather than limit the invention, and that those skilled in the art will be able to design many alternative embodiments without departing from the scope of the appended claims.

In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. Expressions such as "at least one of" when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A surgical robotic system (100) for use in an intraocular procedure, comprising:
- a surgical arm (80) comprising a movable arm part (82), wherein the movable arm part (82) comprises an end effector for holding a surgical instrument (119), wherein the surgical instrument (119) comprises a grasping device (122) for grasping a retinal surface structure (240);
- a sensor (30) configured to attach to or integrate into an intraocular part of the surgical instrument (119), wherein the sensor (30) is configured to generate sensor data (32) which is indicative of a distance between the grasping device (122) and a retinal surface;
- an actuator subsystem (60) configured to actuate the movable arm part;
- a processor subsystem (40) configured to control the actuator subsystem (60) to control a pose of the surgical instrument (119) during the intraocular procedure, wherein the processor subsystem (40) is further configured to:
- when a grasping mode is activated, control the actuator subsystem (60) to automatically adjust the pose of the surgical instrument (119) to position (300) the grasping device (122) at a predetermined distance (D_{T}) to the retinal surface structure, and
- after reaching the predetermined distance to the retinal surface structure, operate the grasping device (122) of the surgical instrument (119) to automatically grasp (310) the retinal surface structure.

2. The surgical robotic system (100) according to claim 1, wherein the processor subsystem (40) is configured to activate the grasping mode and/or operate the grasping device (122) in response to a user command.

3. The surgical robotic system (100) according to claim 1 or 2, wherein the processor subsystem (40) is configured to generate and output sensory perceptible feedback which characterizes one or more operational parameters associated with the grasping mode.

4. The surgical robotic system (100) according to any one of claims 1 to 3, wherein the processor subsystem (40) is configured to, after grasping the retinal surface structure (240) with the grasping device (122), control the actuator subsystem (60) to perform a post-grasp movement (320) with the grasping device (122), for example a retreating movement.

5. The surgical robotic system (100) according to any one of claims 1 to 4, wherein the processor subsystem (40) is configured to, before operating the grasping device (122) to grasp the retinal surface structure, control the actuator subsystem (60) to perform a pre-grasp movement (330) with the grasping device (122).

6. The surgical robotic system (100) according to claim 5, wherein the pre-grasp movement comprises a rotation (330) to adjust an orientation of the surgical instrument (119) in relation to an orientation of the retinal surface structure (240), wherein the processor subsystem (40) is configured to adjust the orientation of the surgical instrument (119) by rotating the surgical instrument (119) about a longitudinal axis of the surgical instrument (119).

7. The surgical robotic system (100) according to claim 6, wherein the processor subsystem (40) is configured to determine the orientation of the retinal surface structure (240) using a geometric model of the retinal surface structure (240) or of the retina, wherein the geometric model is parameterized, and wherein the processor subsystem (40) is configured to determine one or more parameters of the geometric model based on the sensor data (32).

8. The surgical robotic system (100) according to claim 6 or 7, wherein the processor subsystem (40) is configured to determine the orientation of the surgical instrument (119) and/or the orientation of the retinal surface structure (240) based on at least one of:
- the sensor data (32) obtained from the sensor (30);
- extraocular sensor data obtained from an extraocular sensor, such as an optical coherence tomography, OCT, sensor, or a surgical microscope; and
- preoperative data indicative of the orientation of the retinal surface structure.

9. The surgical robotic system (100) according to any one of claims 6 to 8, wherein:
- the grasping device comprises a forceps (122) with arms, wherein the processor subsystem (40) is configured to rotate the surgical instrument (119) to position the arms of the forceps (122) at respective predetermined distances to the retinal surface structure (240), for example by positioning the arms equidistantly to the retinal surface structure; or
- the grasping device (122) comprises a micro vacuum pick, wherein the processor subsystem (40) is configured to rotate the surgical instrument (119) to align an aspiration port of the micro vacuum pick with the retinal surface structure (240).

10. The surgical robotic system (100) according to any one of claims 1 to 9, wherein the processor subsystem (40) is configured to determine a quality of the grasp of the retinal surface structure (240) based on at least one of:
- the sensor data (32) obtained from the sensor (30);
- further sensor data obtained from a further intraoperative sensor, such as an intraoperative impedance or admittance sensor; and
- extraocular sensor data obtained from an extraocular sensor, such as an optical coherence tomography, OCT, sensor, or a surgical microscope.

11. The surgical robotic system (100) according to claim 10, wherein the processor subsystem (40) is configured to trigger a remedial action when the quality of the grasp is determined not to meet a quality threshold, for example by generating and outputting sensory perceptible feedback to the user or by operating the grasping device (122) of the surgical instrument (119) to release and/or to regrasp the retinal surface structure (240).

12. The surgical robotic system (100) according to any one of claims 1 to 11, wherein the sensor data (32) is indicative of periodic physiological movement of a patient, and wherein the processor subsystem (40) is configured to estimate the periodic physiological movement from the sensor data (32) and account or compensate for the periodic physiological movement when controlling the actuator subsystem (60) and/or operating the grasping device (122).

13. The surgical robotic system (100) according to claim 12, wherein the processor subsystem (40) is configured for at least one of:
- control the actuator subsystem (60) to adjust the pose of the surgical instrument (119) to compensate for the periodic physiological movement;
- control the actuator subsystem (60) to, when adjusting the pose of the surgical instrument (119), account for the periodic physiological movement; and
- control the actuator subsystem (60) to operate the grasping device (122) to grasp the retinal surface structure at a time when the periodic physiological movement is estimated to least affect said grasping.

14. The surgical robotic system (100) according to any one of claims 1 to 13, wherein the processor subsystem (40) is configured to control the actuator subsystem (60) to adjust the pose of the surgical instrument (119) using one of:
- closed-loop control in which the sensor data (32) obtained from the sensor (30) is used during movement of the grasping device (122) to update an initial estimate of the distance to the retinal surface structure (240);
- open-loop control in which the initial estimate is not updated during the movement of the grasping device (122).

15. The surgical robotic system (100) according to any one of claims 1 to 14, wherein the sensor (30) is one or a combination of:
- an OCT probe;
- a force-based sensor; and
- an impedance or admittance sensor.

16. A computer program comprising instructions, which when executed by a processor subsystem of a surgical robotic system, cause the processor subsystem to control the surgical robotic system during use in an intraocular procedure, wherein the surgical robotic system comprises:
- a surgical arm comprising a movable arm part, wherein the movable arm part comprises an end effector for holding a surgical instrument, wherein the surgical instrument comprises a grasping device for grasping a retinal surface structure;
- a sensor configured to attach to or integrate into an intraocular part of the surgical instrument, wherein the sensor is configured to generate sensor data which is indicative of a distance between the grasping device and a retinal surface;
- an actuator subsystem configured to actuate the movable arm part;
- the processor subsystem;
wherein the computer program comprises instructions for causing the processor subsystem to:
- control (410) the actuator subsystem to control a pose of the surgical instrument during the intraocular procedure;
- when a grasping mode is activated, control (430) the actuator subsystem to automatically adjust the pose of the surgical instrument to position the grasping device at a predetermined distance to the retinal surface structure; and
- after reaching the predetermined distance to the retinal surface structure, operate (450) the grasping device of the surgical instrument to automatically grasp the retinal surface structure.

## Patentansprüche

1. Ein chirurgisches Robotersystem (100) zur Verwendung bei einem intraokularen Verfahren, umfassend:
- einen chirurgischen Arm (80) mit einem beweglichen Armteil (82), wobei das bewegliche Armteil (82) einen Endeffektor zum Halten eines chirurgischen Instruments (119) umfasst, wobei das chirurgische Instrument (119) eine Greifvorrichtung (122) zum Greifen einer Netzhautoberflächenstruktur (240) umfasst;
- einen Sensor (30), der so konfiguriert ist, dass er an einem intraokularen Teil des chirurgischen Instruments (119) angebracht oder in diesen integriert werden kann, wobei der Sensor (30) so konfiguriert ist, dass er Sensordaten (32) erzeugt, die einen Abstand zwischen der Greifvorrichtung (122) und einer Netzhautoberfläche anzeigen;
- ein Aktuator-Subsystem (60), das so konfiguriert ist, dass es den beweglichen Armteil betätigt;
- ein Prozessor-Subsystem (40), das so konfiguriert ist, dass es das Aktuator-Subsystem (60) steuert zum Steuern einer Position des chirurgischen Instruments (119) während des intraokularen Verfahrens zu steuern, wobei das ProzessorSubsystem (40) ferner zum Folgenden konfiguriert ist:
- wenn ein Greifmodus aktiviert ist, Steuern des Aktuator- Subsystems (60) zum automatischen Anpassen der Position des chirurgischen Instruments (119), um die Greifvorrichtung (122) in einem vorbestimmten Abstand (D_{T}) zur Netzhautoberflächenstruktur zu positionieren, und
- nach dem Erreichen des vorbestimmten Abstands zur Netzhautoberflächenstruktur, Betätigen der Greifvorrichtung (122) des chirurgischen Instruments (119), um die Netzhautoberflächenstruktur automatisch zu greifen (310).

2. Das chirurgische Robotersystem (100) nach Anspruch 1, wobei das Prozessor-Subsystem (40) so konfiguriert ist, dass es den Greifmodus aktiviert und/oder die Greifvorrichtung (122) als Reaktion auf einen Benutzerbefehl betätigt.

3. Das chirurgische Robotersystem (100) nach Anspruch 1 oder 2, wobei das Prozessor-Subsystem (40) so konfiguriert ist, dass es eine sensorisch wahrnehmbare Rückmeldung erzeugt und ausgibt, die einen oder mehrere mit dem Greifmodus verbundene Betriebsparameter kennzeichnet.

4. Das chirurgische Robotersystem (100) nach einem der Ansprüche 1 bis 3, wobei das Prozessor-Subsystem (40) so konfiguriert ist, dass es, nach dem Erfassen der Netzhautoberflächenstruktur (240) mit der Greifvorrichtung (122), das Aktuator-Subsystem (60) zum Ausführen einer nach-Greif-Bewegung (320) mit der Greifvorrichtung (122), beispielsweise eine Rückzugsbewegung, steuert.

5. Das chirurgische Robotersystem (100) nach einem der Ansprüche 1 bis 4, wobei das Prozessor-Subsystem (40) so konfiguriert ist, dass es, vor dem Betätigen der Greifvorrichtung (122) zum Greifen der Netzhautoberflächenstruktur, das Aktuator-Subsystem (60) zum Ausführen einer Vor-Greifbewegung (330) mit der Greifvorrichtung (122) steuert.

6. Das chirurgische Robotersystem (100) nach Anspruch 5, wobei die Vor-Greifbewegung eine Drehung (330) umfasst, um eine Ausrichtung des chirurgischen Instruments (119) in Bezug auf eine Ausrichtung der Netzhautoberflächenstruktur (240) anzupassen, wobei das Prozessor-Subsystem (40) so konfiguriert ist, dass es die Ausrichtung des chirurgischen Instruments (119) durch Drehen des chirurgischen Instruments (119) um eine Längsachse des chirurgischen Instruments (119) einstellt.

7. Das chirurgische Robotersystem (100) nach Anspruch 6, wobei das Prozessor-Subsystem (40) so konfiguriert ist, dass es die Ausrichtung der Netzhautoberflächenstruktur (240) unter Verwendung eines geometrischen Modells der Netzhautoberflächenstruktur (240) oder der Netzhaut bestimmt, wobei das geometrische Modell parametrisiert ist und wobei das Prozessor-Subsystem (40) so konfiguriert ist, dass es einen oder mehrere Parameter des geometrischen Modells auf der Grundlage der Sensordaten (32) bestimmt.

8. Das chirurgische Robotersystem (100) nach Anspruch 6 oder 7, wobei das Prozessor-Subsystem (40) so konfiguriert ist, dass es die Ausrichtung des chirurgischen Instruments (119) und/oder die Ausrichtung der Netzhautoberflächenstruktur (240) auf der Grundlage mindestens eines des Folgenden bestimmt:
- die vom Sensor (30) erhaltenen Sensordaten (32);
- extraokulare Daten, die von einem extraokularen Sensor, wie beispielsweise einem Sensor für optische Kohärenztomographie (OCT) oder einem Operationsmikroskop, erfasst werden; und
- präoperative Daten, die die Ausrichtung der Netzhautoberfläche anzeigen.

9. Das chirurgische Robotersystem (100) nach einem der Ansprüche 6 bis 8, wobei:
- die Greifvorrichtung eine Zange (122) mit Armen umfasst, wobei das Prozessorsubsystem (40) so konfiguriert ist, dass es das chirurgische Instrument (119) dreht, um die Arme der Zange (122) in jeweils vorbestimmten Abständen zur Netzhautoberflächenstruktur (240) zu positionieren, beispielsweise indem die Arme in gleichem Abstand zur Netzhautoberflächenstruktur positioniert werden; oder
- die Greifvorrichtung (122) einen Mikro-Vakuumgreifer umfasst, wobei das Prozessor-Subsystem (40) so konfiguriert ist, dass es das chirurgische Instrument (119) dreht, um ein Ansauganschluss des Mikro-Vakuumgreifers mit der Netzhautoberflächenstruktur (240) auszurichten.

10. Das chirurgische Robotersystem (100) nach einem der Ansprüche 1 bis 9, wobei das Prozessor-Subsystem (40) so konfiguriert ist, dass es eine Qualität des Greifens der Netzhautoberflächenstruktur (240) auf der Grundlage mindestens eines des Folgenden bestimmt:
- den vom Sensor (30) erhaltenen Sensordaten (32);
- weiteren Sensordaten, die von einem weiteren intraoperativen Sensor, wie beispielsweise einem intraoperativen Impedanz- oder Admittanzsensor, erhalten wurden; und
- extraokularen Sensordaten, die von einem extraokularen Sensor, wie beispielsweise einem optischen Kohärenztomographie-Sensor (OCT-Sensor) oder einem Operationsmikroskop, erhalten wurden.

11. Das chirurgische Robotersystem (100) nach Anspruch 10, wobei das Prozessor-Subsystem (40) so konfiguriert ist, dass es eine Abhilfemaßnahme auslöst, wenn festgestellt wird, dass die Qualität des Greifens einen Qualitätsschwellenwert nicht erfüllt, beispielsweise durch Erzeugen und Ausgeben einer sensorisch wahrnehmbaren Rückmeldung an den Benutzer oder durch Betätigen der Greifvorrichtung (122) des chirurgischen Instruments (119), um die Netzhautoberflächenstruktur (240) freizugeben und/oder erneut zu greifen.

12. Das chirurgische Robotersystem (100) nach einem der Ansprüche 1 bis 11, wobei die Sensordaten (32) eine periodische physiologische Bewegung eines Patienten anzeigen und wobei das Prozessor-Subsystem (40) so konfiguriert ist, dass es die periodische physiologische Bewegung aus den Sensordaten (32) schätzt und die periodische physiologische Bewegung bei der Steuerung des Aktuator-Subsystems (60) und/oder beim Betreiben der Greifvorrichtung (122) berücksichtigt oder kompensiert.

13. Das chirurgische Robotersystem (100) nach Anspruch 12, wobei das Prozessor-Subsystem (40) für mindestens eines des Folgenden konfiguriert ist:
- das Aktuator-Subsystem (60) zu steuern, zum Anpassen der Position des chirurgischen Instruments (119), um die periodische physiologische Bewegung auszugleichen;
- das Aktuator-Subsystem (60) zu steuern, zum Berücksichtigen der periodische physiologische Bewegung bei der Einstellung der Position des chirurgischen Instruments (119); und
- das Aktuator-Subsystem (60) zu steuern, zum Betätigen der Greifvorrichtung (122), zum Greifen der Netzhautoberflächenstruktur zu einem Zeitpunkt, zu dem die periodische physiologische Bewegung das Greifen voraussichtlich am wenigsten beeinträchtigt.

14. Das chirurgische Robotersystem (100) nach einem der Ansprüche 1 bis 13, wobei das Prozessor-Subsystem (40) konfiguriert ist zum Steuern des Aktuator-Subsystems (60) zum Anpassen der Position des chirurgischen Instruments (119) unter Verwendung einer des Folgenden:
- einen geschlossenen Regelkreis, in dem die vom Sensor (30) erhaltenen Sensordaten (32) während einer Bewegung der Greifvorrichtung (122) verwendet werden, um eine anfängliche Schätzung der Entfernung zur Netzhautoberflächenstruktur (240) zu aktualisieren;
- eine offenen Regelkreis, in dem die anfängliche Schätzung während der Bewegung der Greifvorrichtung (122) nicht aktualisiert wird.

15. Das chirurgische Robotersystem (100) nach einem der Ansprüche 1 bis 14, wobei der Sensor (30) einer oder eine Kombination aus:
- einer OCT-Sonde;
- einem kraftbasierten Sensor; und
- einem Impedanz- oder Admittanzsensor
ist.

16. Ein Computerprogramm, das Anweisungen umfasst, die, wenn sie von einem Prozessor-Subsystem eines chirurgischen Robotersystems ausgeführt werden, bewirken, dass das Prozessor-Subsystem das chirurgische Robotersystem während der Verwendung in einem intraokularen Verfahren steuert, wobei das chirurgische Robotersystem umfasst:
- einen chirurgischen Arm, der einen beweglichen Armteil umfasst, wobei der bewegliche Armteil einen Endeffektor zum Halten eines chirurgischen Instruments umfasst, wobei das chirurgische Instrument eine Greifvorrichtung zum Greifen einer Netzhautoberflächenstruktur umfasst;
- einen Sensor, der so konfiguriert ist, dass er an einem intraokularen Teil des chirurgischen Instruments angebracht oder in dieses integriert werden kann, wobei der Sensor so konfiguriert ist, dass er Sensordaten erzeugt, die einen Abstand zwischen der Greifvorrichtung und einer Netzhautoberfläche anzeigen;
- ein Aktuator-Subsystem, das zum Betätigen des beweglichen Armteils konfiguriert ist;
- das Prozessor-Subsystem;
wobei das Computerprogramm Anweisungen umfasst, die das Prozessor-Subsystem dazu veranlassen:
- das Aktuator-Subsystem so zu steuern (410), dass es eine Position des chirurgischen Instruments während des intraokularen Verfahrens steuert;
- wenn ein Greifmodus aktiviert ist, das Aktuator-Subsystem so zu steuern (430), dass die Position des chirurgischen Instruments automatisch angepasst wird, um die Greifvorrichtung in einem vorbestimmten Abstand zur Netzhautoberflächenstruktur zu positionieren; und
- nach Erreichen des vorbestimmten Abstands zur Netzhautoberflächenstruktur die Greifvorrichtung des chirurgischen Instruments zu betätigen (450), um automatisch die Netzhautoberflächenstruktur zu greifen.

## Revendications

1. Un système robotique chirurgical (100) destiné à être utilisé dans une procédure intraoculaire, comprenant :
- un bras chirurgical (80) comprenant une partie de bras mobile (82), dans lequel la partie de bras mobile (82) comprend un effecteur terminal pour maintenir un instrument chirurgical (119), dans lequel l'instrument chirurgical (119) comprend un dispositif de préhension (122) pour saisir une structure de surface rétinienne (240) ;
- un capteur (30) configuré pour être fixé à ou intégré dans une partie intraoculaire de l'instrument chirurgical (119), dans lequel le capteur (30) est configuré pour générer des données de capteur (32) qui indiquent une distance entre le dispositif de préhension (122) et une surface rétinienne ;
- un sous-système actionneur (60) configuré pour actionner la partie de bras mobile ;
- un sous-système processeur (40) configuré pour commander le sous-système actionneur (60) afin de contrôler la position de l'instrument chirurgical (119) pendant la procédure intraoculaire, dans lequel le sous-système processeur (40) est en outre configuré pour :
- lorsqu'un mode de préhension est activé, commander le sous-système actionneur (60) afin d'ajuster automatiquement la position de l'instrument chirurgical (119) pour positionner (300) le dispositif de préhension (122) à une distance prédéterminée (D_{T}) de la structure de surface rétinienne, et
- après avoir atteint la distance prédéterminée par rapport à la structure de surface rétinienne, actionner le dispositif de préhension (122) de l'instrument chirurgical (119) pour saisir automatiquement (310) la structure de surface rétinienne.

2. Le système robotique chirurgical (100) selon la revendication 1, dans lequel le sous-système processeur (40) est configuré pour activer le mode de préhension et/ou actionner le dispositif de préhension (122) en réponse à une commande de l'utilisateur.

3. Le système robotique chirurgical (100) selon la revendication 1 ou 2, dans lequel le sous-système processeur (40) est configuré pour générer et produire un feedback perceptible par les sens qui caractérise un ou plusieurs paramètres opérationnels associés au mode de préhension.

4. Le système robotique chirurgical (100) selon l'une quelconque des revendications 1 à 3, dans lequel le sous-système processeur (40) est configuré pour, après avoir saisi la structure de surface rétinienne (240) avec le dispositif de préhension (122), commander le sous-système actionneur (60) afin d'effectuer un mouvement post-préhension (320) avec le dispositif de préhension (122), par exemple un mouvement de retrait.

5. Le système robotique chirurgical (100) selon l'une quelconque des revendications 1 à 4, dans lequel le sous-système processeur (40) est configuré pour, avant d'actionner le dispositif de préhension (122) afin de saisir la structure de surface rétinienne, commander le sous-système actionneur (60) afin d'effectuer un mouvement de pré-préhension (330) avec le dispositif de préhension (122).

6. Le système robotique chirurgical (100) selon la revendication 5, dans lequel le mouvement de pré-préhension comprend une rotation (330) pour ajuster une orientation de l'instrument chirurgical (119) par rapport à une orientation de la structure de surface rétinienne (240), dans lequel le sous-système processeur (40) est configuré pour ajuster l'orientation de l'instrument chirurgical (119) en faisant tourner l'instrument chirurgical (119) autour d'un axe longitudinal de l'instrument chirurgical (119).

7. Le système robotique chirurgical (100) selon la revendication 6, dans lequel le sous-système processeur (40) est configuré pour déterminer l'orientation de la structure de surface rétinienne (240) à l'aide d'un modèle géométrique de la structure de surface rétinienne (240) ou de la rétine, dans lequel le modèle géométrique est paramétré, et dans lequel le sous-système processeur (40) est configuré pour déterminer un ou plusieurs paramètres du modèle géométrique sur la base des données du capteur (32).

8. Le système robotique chirurgical (100) selon la revendication 6 ou 7, dans lequel le sous-système processeur (40) est configuré pour déterminer l'orientation de l'instrument chirurgical (119) et/ou l'orientation de la structure de surface rétinienne (240) sur la base d'au moins l'un de:
- les données du capteur (32) obtenues à partir du capteur (30) ;
- les données du capteur extra-oculaire obtenues à partir d'un capteur extra-oculaire, tel qu'un capteur de tomographie en cohérence optique (TCO) ou un microscope chirurgical ; et
- les données préopératoires indiquant l'orientation de la structure de la surface rétinienne.

9. Le système robotique chirurgical (100) selon l'une quelconque des revendications 6 à 8, dans lequel :
- le dispositif de préhension comprend un forceps (122) avec des bras, dans lequel le sous-système processeur (40) est configuré pour faire tourner l'instrument chirurgical (119) afin de positionner les bras du forceps (122) à des distances prédéterminées respectives par rapport à la structure de surface rétinienne (240), par exemple en positionnant les bras à égale distance de la structure de surface rétinienne ; ou
- le dispositif de préhension (122) comprend une micro-pince à vide, dans lequel le sous-système processeur (40) est configuré pour faire tourner l'instrument chirurgical (119) afin d'aligner un port d'aspiration de la micro-pince à vide avec la structure de surface rétinienne (240).

10. Le système robotique chirurgical (100) selon l'une quelconque des revendications 1 à 9, dans lequel le sous-système processeur (40) est configuré pour déterminer une qualité de la préhension de la structure de surface rétinienne (240) sur la base d'au moins l'un de :
- les données de capteur (32) obtenues à partir du capteur (30) ;
- données de capteur supplémentaires obtenues à partir d'un autre capteur intra-opératoire, tel qu'un capteur d'impédance ou d'admittance intra-opératoire ; et
- les données du capteur extra-oculaire obtenues à partir d'un capteur extra-oculaire, tel qu'un capteur de tomographie en cohérence optique (TCO) ou un microscope chirurgical.

11. Le système robotique chirurgical (100) selon la revendication 10, dans lequel le sous-système processeur (40) est configuré pour déclencher une action corrective lorsque la qualité de la préhension est jugée inférieure à un seuil de qualité, par exemple en générant et en produisant un feedback perceptible par les sens à l'utilisateur ou en actionnant le dispositif de préhension (122) de l'instrument chirurgical (119) pour relâcher et/ou re-saisir la structure de surface rétinienne (240).

12. Le système robotique chirurgical (100) selon l'une quelconque des revendications 1 à 11, dans lequel les données du capteur (32) sont indicatives d'un mouvement physiologique périodique d'un patient, et dans lequel le sous-système processeur (40) est configuré pour estimer le mouvement physiologique périodique à partir des données du capteur (32) et pour tenir compte ou compenser le mouvement physiologique périodique lors de la commande du sous-système actionneur (60) et/ou de l'actionnement du dispositif de préhension (122).

13. Le système robotique chirurgical (100) selon la revendication 12, dans lequel le sous-système processeur (40) est configuré pour au moins l'une de :
- commander le sous-système actionneur (60) afin d'ajuster la position de l'instrument chirurgical (119) pour compenser le mouvement physiologique périodique ;
- commander le sous-système actionneur (60) afin de tenir compte du mouvement physiologique périodique lors de l'ajustement de la position de l'instrument chirurgical (119) ; et
- commander le sous-système actionneur (60) pour actionner le dispositif de préhension (122) afin de saisir la structure de surface rétinienne à un moment où le mouvement physiologique périodique est estimé avoir le moins d'effet sur ladite action de saisir.

14. Le système robotique chirurgical (100) selon l'une quelconque des revendications 1 à 13, dans lequel le sous-système processeur (40) est configuré pour commander le sous-système actionneur (60) afin d'ajuster la position de l'instrument chirurgical (119) en utilisant l'un de :
- une commande en boucle fermée dans laquelle les données de capteur (32) obtenues à partir du capteur (30) sont utilisées pendant le mouvement du dispositif de préhension (122) pour mettre à jour une estimation initiale de la distance par rapport à la structure de surface rétinienne (240) ;
- une commande en boucle ouverte dans laquelle l'estimation initiale n'est pas mise à jour pendant le mouvement du dispositif de préhension (122).

15. Le système robotique chirurgical (100) selon l'une quelconque des revendications 1 à 14, dans lequel le capteur (30) est l'un ou une combinaison de :
- une sonde TCO ;
- un capteur basé sur la force ; et
- un capteur d'impédance ou d'admittance.

16. Un programme informatique comprenant des instructions qui, lorsqu'elles sont exécutées par un sous-système processeur d'un système robotique chirurgical, amènent le sous-système processeur à commander le système robotique chirurgical pendant son utilisation dans une procédure intraoculaire, dans lequel le système robotique chirurgical comprend :
- un bras chirurgical comprenant une partie de bras mobile, dans lequel la partie de bras mobile comprend un effecteur terminal pour maintenir un instrument chirurgical, dans lequel l'instrument chirurgical comprend un dispositif de préhension pour saisir une structure de surface rétinienne ;
- un capteur configuré pour être fixé à ou intégré dans une partie intraoculaire de l'instrument chirurgical, dans lequel le capteur est configuré pour générer des données de capteur qui indiquent une distance entre le dispositif de préhension et une surface rétinienne ;
- un sous-système actionneur configuré pour actionner la partie de bras mobile ;
- le sous-système processeur ;
dans lequel le programme informatique comprend des instructions pour amener le sous-système processeur à :
- commander (410) le sous-système actionneur afin de contrôler une position de l'instrument chirurgical pendant la procédure intraoculaire ;
- lorsqu'un mode de préhension est activé, commander (430) le sous-système actionneur afin d'ajuster automatiquement la position de l'instrument chirurgical pour positionner le dispositif de préhension à une distance prédéterminée de la structure de surface rétinienne ; et
- après avoir atteint la distance prédéterminée par rapport à la structure de surface rétinienne, actionner (450) le dispositif de préhension de l'instrument chirurgical pour saisir automatiquement la structure de surface rétinienne.
